# EUROPEAN PATENT APPLICATION

(11) **EP 4 434 973 A1**
(43) Date of publication of application: **25.09.2024**
(21) Application number: 23162993.2
(22) Date of filing: 20.03.2023
(51) Int. Cl.: C07D 251/26

(54) **TAG FOR LIQUID-PHASE PEPTIDE SYNTHESIS**

(71) Applicant: CordenPharma International GmbH, 68723 Plankstadt (DE)
(72) Inventor: GIRAUD, Matthieu, 68723 Plankstadt (DE); ALBERICIO, Fernando, 68723 Plankstadt (DE); MARDER, Oleg, 68723 Plankstadt (DE); HELLRIEGEL, Laura, 68723 Plankstadt (DE); HÜTTENSCHMIDT, Mareike, 68723 Plankstadt (DE); HOGENKAMP, Fabian, 68723 Plankstadt (DE); KRAMER, Tim, 68723 Plankstadt (DE)
(74) Representative: Schulz Junghans Patentanwälte PartGmbB

(57) **Abstract**

The present invention relates compound according to formula (I) 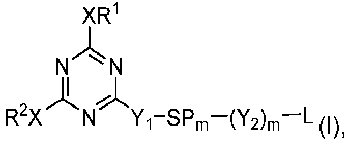 wherein,
X is selected from the group consisting of -O-(CH₂)ₙ-, -(NMe)-(CH₂)ₙ-, or -S-(CH₂)ₙ-, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an alkyl substituted C₆-aromatic compound,
Y is a heteroatom, particularly selected from the group consisting of -O- and -NH-,
L is a linker for peptide synthesis and
m is 0 or 1, particularly m is 1.

Additionally, the present invention relates to an intermediate of formula (II) 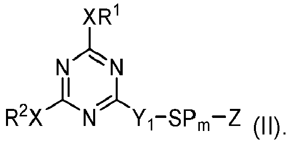 wherein,
Z is selected from -OH, -NH₂.

## Description

### Field

The present invention relates to a novel tag for liquid-phase peptide synthesis.

### Background

The invention of Solid Phase Peptide Synthesis (SPPS) by Merrifield in 1963 revolutionized peptide chemistry by implementation of a resin, which allows the coupling and deprotection reactions to be carried out in a heterogenous system and therefore removal of the excess reagents and side products by simple washing steps (Merrifield, R. B., J. Am. Chem. Soc. 1963, 85 (14), 2149-2154). Although efficient SPPS demands excess of reagents and large amounts of solvent, the commonly used Fmoc-tBu strategy can be easily scaled-up and automated for large-scale production of peptides. However, in the context of green chemistry the large amount of waste and the use of hazardous solvents, such as DMF, NMP and DCM, in SPPS should be addressed. In contrast, Liquid-Phase Peptide Synthesis (LPPS) represents an approach which is compatible with these demands, since the reactions are carried out in solution using soluble tags attached to the growing peptide chain (for review see: Albericio, F. et. al. Chem. Rev. 2022, 16, 13516-13546). LPPS can be carried out using less solvent and excess of reagents, while also providing the possibility of implementing green solvents, such as tetrahydrofuran (Chiba, K. Tamaki, H. et. Al. Org. Process Res. Dev. 2019, 23, 11, 2576-2581) and cyclopentyl methyl ether (Kubota, H. et. al. Molecules 2021, 26 (12), 3497). Starting from early PEG-based approaches by Mutter and Bayer (Bayer, E. and Mutter, M., Nature 1972, 237, 512-513; Mutter, M. and Bayer, E., Angew. Chem., Int. Ed. Engl. 1974, 13, 88-89), Tamaki et. al. (Tamaki, H. et. al. Bull. Chem. Soc. Jpn. 2001, 74, 733-738) invented substituted hydrophobic benzyl alcohols (HABs) for tag-assisted LPPS, which have been improved by different groups (e.g.: Sunazuka, T. et. al. Tetrahedron 2011, 67, 6633-6643 and Takahashi, D. et. al. Angew. Chem., Int. Ed. 2017, 56, 7803-7807). These works successfully used soluble HAB-tags for the synthesis of small to medium sized peptides using the Fmoc approach, while usually achieving purification of the desired peptide by precipitation or membrane-assisted filtration and in some cases aqueous work-up procedures.

Based on the above-mentioned state of the art, the objective of the present invention is to provide means and methods for a novel tag for peptide synthesis in solution.This objective is attained by the subject-matter of the independent claims of the present specification, with further advantageous embodiments described in the dependent claims, examples, figures and general description of this specification.

### Summary of the Invention

A first aspect of the invention relates to a compound according to formula (I) 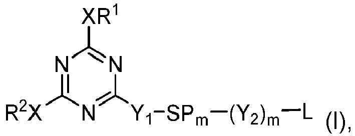 wherein,
X is selected from the group consisting of -O-(CH₂)ₙ-,-NH-(CH₂)ₙ-, -(NMe)-(CH₂)ₙ-, or -S-(CH₂)ₙ-, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an alkyl substituted C₆-aromatic compound,
Y is a heteroatom, particularly selected from the group consisting of -O- and -NH-,
L is a linker for peptide synthesis, and
m is 0 or 1, particularly m is 1.

A second aspect of the invention relates to an intermediate according to formula (II) 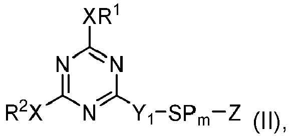 wherein,
X is selected from the group consisting of -O-(CH₂)ₙ-,-NH-(CH₂)ₙ, -(NMe)-(CH₂)ₙ-, or -S-(CH₂)ₙ-, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an alkyl substituted C₆-aromatic compound,
Y is a heteroatom, particularly selected from the group consisting of -O- and -NH-,
Z is selected from -OH, -NH₂, and
wherein m is 0 or 1, particularly m is 1.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *linker* in the context of the present specification relates to a molecule used in Fmoc-Solid Phase Peptide Synthesis (SPPS), which connects the resin on which the SPPS is performed and the forming peptide. The linker may be separated from the peptide through a spacer. The linker is cleaved from the peptide once the desired peptide length is reached.

The term *spacer* in the context of the present specification relates to a flexible hydrophobic compound consisting of hydrocarbon atoms connecting a linker molecule and the tag core.

The term *tag* in the context of the present specification relates to a molecule on which a peptide sequence is formed. A tag is usually attached at the N- or C-terminus of the forming peptide.

The formulae of the present specification follow the convention of organic chemistry to not show hydrogen atoms on carbon scaffolds. Carbon is tetravalent and bonds not shown are assumed to be hydrogen unless shown otherwise. Hydrogen can be exchanged for deuterium without changing the bulk chemical properties of the molecule; however, in the case of dye or drug molecules, the exchange of hydrogen for deuterium may lead to changes in the spectral properties or receptor interactions of the molecule. Unless explicitly stated otherwise herein, the disclosure of a formula showing, explicitly or implicitly by the convention restated in the first sentence of this paragraph, encompasses molecules in which one or several of the hydrogen atoms are exchanged for deuterium.

The term *saturated alkyl* in the context of the present specification relates to a saturated linear or branched hydrocarbon.

The term *unsaturated alkyl* in the context of the present specification relates to a linear or branched hydrocarbon, wherein one or more carbon-carbon bonds may be unsaturated.

The term *alkyl* in the context of the present specification relates to a saturated linear, branched or cyclic hydrocarbon consisting of a designated number of carbon atoms. Examples of alkyl residuesinclude but are not limited to n-butyl, iso-butyl, n-pentyl, 2-methylbutyl, 3-methylbutyl, 1,1-dimethylpropyl, 1,2-dimethylpropyl, 1,2-dimethylpropyl, cyclo-pentyl, cyclo-hexyl, methyl-cyclo-pentyl, methyl cyclohexanyl, heptyl, decanyl, dodecyl. In certain embodiments, a C₅ alkyl is a pentyl or cyclopentyl moiety and a C₆ alkyl is a hexyl or cyclohexyl moiety and a C₇ alkyl is a heptyl or cycloheptyl, which also applies to higher ranking alkyls as generally understood by a person skilled in the art.

The term alkyl substituted C₆-*aryl* in the context of the present specification relates to a cyclic aromatic C₆ hydrocarbon. The C₆-aryl may be further substituted with in one or several carbon atoms by groups selected from saturated or unsaturated hydrocarbons (alkyl in their broadest sense). Examples of include but are not limited to phenyl, benzyl, xylylenyl and toluenyl.

### Detailed Description of the Invention

A first aspect of the invention relates to a compound according to formula (I) 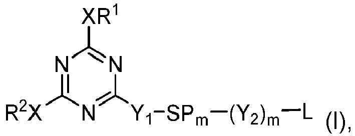 wherein,
X is selected from the group consisting of -O-(CH₂)ₙ-, -NH-(CH₂)ₙ-, -(NMe)-(CH₂)ₙ-, or -S-(CH₂)ₙ-, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an C₆-aromatic compound,
Y is a heteroatom, particularly selected from the group consisting of -O- and -NH-,
L is a linker for peptide synthesis, and
m is 0 or 1, particularly m is 1.

The compound is used as a tag to perform peptide elongation in greener solvents compared to SPPS. Furthermore, the new tags allow a simple work-up of the coupling reactions by simple phase separation. The resulting tag conjugated peptide remains in the organic phase and can be used directly for the next coupling step without any further purification step. The synthesis of the desired tag is established from readily available and cheap starting materials, providing an affordable molecule for LPPS.

In certain embodiments, X is selected from -O-(CH₂)ₙ-, -S-(CH₂)ₙ-, wherein n is 0 to 4.

In certain embodiments, X is selected from -O-(CH₂)ₙ-, wherein n is 0 to 4.

In certain embodiments, n is 0.

In certain embodiments, R¹ and R² are independently from each other a branched or substituted C₁₁-C₂₅ alkyl.

In certain embodiments, R¹ and R² are independently selected from the group consisting of C₁₃-C₂₅ alkyl.

In certain embodiments R¹ is selected from the group consisting of C₁₇-C₂₅ alkyl.

In certain embodiments, R¹ is selected from the group consisting of C₂₁-C₂₅ alkyl.

In certain embodiments, R² is selected from the group consisting of C₁₇-C₂₅ alkyl.

In certain embodiments, R² is selected from the group consisting of C₂₁-C₂₅ alkyl.

In certain embodiments, R¹ and R² are a branched or substituted C₁₁-C₂₅ alkyl.

In certain embodiments, R¹ and R² are selected from the group consisting of C₁₃-C₂₅ alkyl.

In certain embodiments, R¹ and R² are selected from the group consisting of C₁₇-C₂₅ alkyl.

In certain embodiments, R¹ and R² are selected from the group consisting of C₂₁-C₂₅ alkyl.

In certain embodiments, Y₁ is -NH- and Y₂ is -NH- or -O.

In certain embodiments, Y₁ is -NH- and Y₂ is -NH-.

In certain embodiments, SP is selected from the group consisting of saturated C₄-C₈ alkyl, unsaturated C₄-C₈ alkyl and substituted C₆-aromatic compound.

In certain embodiments, SP is selected from the group consisting of saturated C₄-C₈ alkyl and an alkyl substituted C₆-aromatic compound.

In certain embodiments, SP is selected from the group consisting of saturated C₅-C₇ alkyl and an alkyl substituted C₆-aromatic compound.

In certain embodiments, SP is selected from the group consisting of saturated C₅-C₇ alkyl.

In certain embodiments, the linker L selected from the group consisting of Rink, Sieber, Trityl, Ramage, Wang, Sasrin, HMPB, 2-chlorotrityl and MBHA.

In certain embodiments, the linker L is selected from the group consisting of Rink, Sieber and Trityl.

The tag can be used in peptide synthesis which in comparison to conventional Solid Phase Peptide Synthesis is performed in greener solvents and less amount of solvent making this approach a more environmentally friendly and cost-effective approach to synthesise peptides.

Furthermore, the use of the tag in peptide synthesis does not require additional purification steps which results higher peptide yields compared to conventional peptide synthesis.

A second aspect of the invention relates to an intermediate according to formula (II) 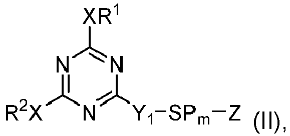 wherein,
X is selected from the group consisting of -O-(CH₂)ₙ-, -NH-(CH₂)ₙ-, -(NMe)-(CH₂)ₙ-, or -S-(CH₂)ₙ-, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an alkyl substituted C₆-aromatic compound an alkyl substituted C₆-aromatic compound,
Y is a heteroatom, particularly selected from the group consisting of -O- and -NH-, Z is selected from -OH and -NH₂, and
m is 0 or 1, particularly m is 1.

As for the definitions of formula (II), it is referred to the definitions of formula (I) of the first aspect of the invention.

In certain embodiments, X is selected from -O-(CH₂)ₙ-, -S-(CH₂)ₙ-, wherein n is 0 to 4.

In certain embodiments, X is selected from -O-(CH₂)ₙ-, wherein n is 0 to 4.

In certain embodiments, n is 0.

In certain embodiments, R¹ and R² are independently from each other a branched or substituted C₁₁-C₂₅ alkyl.

In certain embodiments, R¹ and R² are independently selected from the group consisting of C₁₃-C₂₅ alkyl.

In certain embodiments R¹ is selected from the group consisting of C₁₇-C₂₅ alkyl.

In certain embodiments, R¹ is selected from the group consisting of C₂₁-C₂₅ alkyl.

In certain embodiments, R² is selected from the group consisting of C₁₇-C₂₅ alkyl.

In certain embodiments, R² is selected from the group consisting of C₂₁-C₂₅ alkyl.

In certain embodiments, R¹ and R² are a branched or substituted C₁₁-C₂₅ alkyl.

In certain embodiments, R¹ and R² are selected from the group consisting of C₁₃-C₂₅ alkyl.

In certain embodiments, R¹ and R² are selected from the group consisting of C₁₇-C₂₅ alkyl.

In certain embodiments, R¹ and R² are selected from the group consisting of C₂₁-C₂₅ alkyl.

In certain embodiments, Y₁ is -NH- and Z is -NH- or -O.

In certain embodiments, Y₁ is -NH- and Z is -NH-.

In certain embodiments, SP is selected from the group consisting of saturated C₄-C₈ alkyl, unsaturated C₄-C₈ alkyl and substituted C₆-aromatic compound, in particular the substituted C₆-aromatic compound is an alkyl substituted C₆-aromatic compound.

In certain embodiments, SP is selected from the group consisting of saturated C₄-C₈ alkyl and alkyl substituted C₆-aromatic compound.

In certain embodiments, SP is selected from the group consisting of saturated C₅-C₇ alkyl and an alkyl substituted C₆-aromatic compound.

In certain embodiments, SP is selected from the group consisting of saturated C₅-C₇ alkyl.

The invention is further illustrated by the following examples, from which further embodiments and advantages can be drawn. These examples are meant to illustrate the invention but not to limit its scope.

### Examples

### Example 1: Synthesis of the tag

### Preparation of 2-chloro-4,6-bis(heptadecan-9-yloxy)-1,3,5-triazine

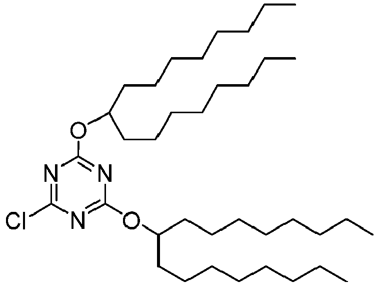

Under nitrogen atmosphere, heptadecan-9-ol (48.68 g, 3.50 eq, 189.8 mmol) was dissolved in dry, stabilised THF (500 mL) and sodium hydride (11 g, 60% Wt, 5.00 eq, 271.1 mmol) was added in portions at 4°C for 10 min. After complete addition the reaction was allowed to warm to room temperature and meanwhile was stirred for 1 h. Then the 2,4,6-trichloro-1,3,5-triazine (10.000 g, 1.00 eq, 54.23 mmol) was added in portions for 10 min and stirred at 40°C for 40 h.

The reaction was quenched in an ice-bath with water (-100 mL) for 15 min and was diluted with ethyl acetate (200 mL). The pH was adjusted to pH 5 by adding 1 M HCl. The mixture was transferred to a separation funnel and was extracted. The combined organic phases were washed with brine (100 mL) and dried over MgSO₄. The solvent was removed under reduced pressure, obtaining the crude (16.24 g, 26.0 mmol, 48.0%) as a white wax-like powder.

The crude was further purified by column chromatography (1000 g, SiO₂; EtOAc/n-heptane, 1 :50), obtaining the product as a light-yellow oil.

### Preparation of N1-(4,6-bis(heptadecan-9-yloxy)-1,3,5-triazin-2-yl)hexane-1,6-diamine

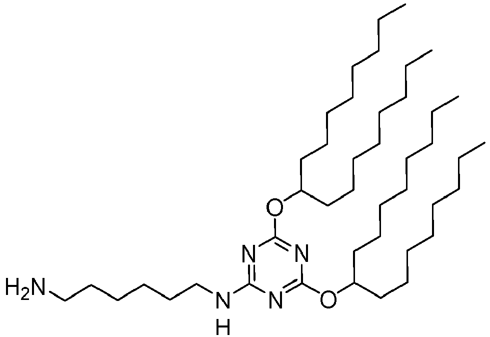

Hexane-1,6-diamine (10.236 g, 5.00 eq, 88.079 mmol) was dissolved in toluene (200 mL) and stirred for 1 h at room temperature. 2-chloro-4,6-bis(heptadecan-9-yloxy)-1,3,5-triazine (11.000 g, 1.00 eq, 17.616 mmol) was dissolved in toluene (100 mL) and added to the reaction. Then diisopropylethylamine (17 g, 23 mL, 7.6 eq, 0.13 mol) was added and the reaction stirred for 48 h at 80 °C.

The solvent was removed under reduced pressure. The mixture was diluted in 200 mL EtOAc and was transferred to a separation funnel and was extracted three times with water (100 mL) and with brine 50 mL. The solvent was removed under reduced pressure, obtaining the crude (9.85 g, 13.98 mmol, 79.4%) as a yellowish oil.

The product was used without further purification.

### Preparation of (9H-fluoren-9-yl)methyl ((4-(2-((6-((4,6-bis(heptadecan-9-yloxy)-1,3,5-triazin-2-yl)amino)hexyl)amino)-2-oxoethoxy)phenyl)(2,4-dimethoxyphenyl)methyl)carbamate

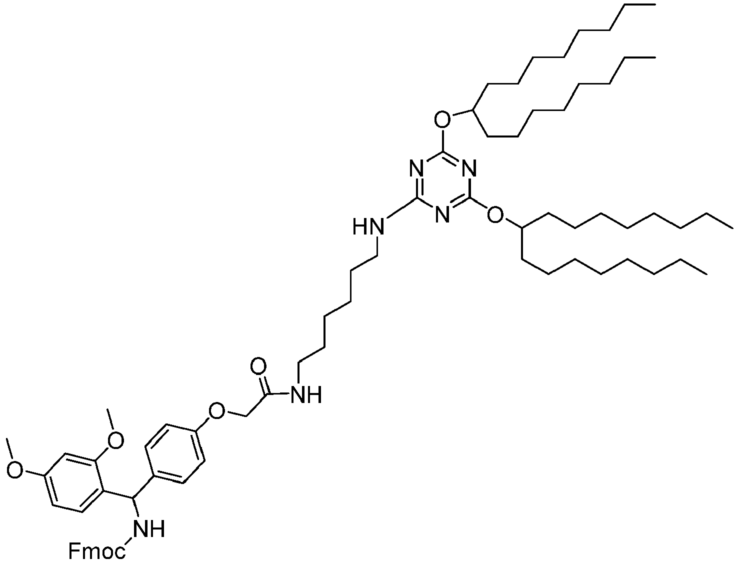

Fmoc-Rink-Linker (15.325 g, 2.00 eq, 28.401 mmol) and HOBt (4.8612 g, 2.00 eq, 28.401 mmol) were dissolved in DCM (250 mL) and EDC HCl (5.500 g, 99% Wt, 2.00 eq, 28.401 mmol) was added in portions over 5 min. After stirring at room temperature for 1 h, the reaction was cooled in an ice-bath (4°C internal) and 4-methylmorpholine (2.8728 g, 3.2 mL, 2.00 eq, 28.401 mmol) was added. Following, N1-(4,6-bis(heptadecan-9-yloxy)-1,3,5-triazin-2-yl)hexane-1,6-diamine (10.000 g, 1.00 eq, 14.201 mmol) was added in portions for 5 min, keeping the internal temperature below 10°C. After complete addition the reaction mixture was allowed to warm to room temperature.

After stirring for 8 h at 40°C the suspension was evaporated to remove the DCM. The crude was diluted with EtOAc and washed three times with water (100 mL) and one time with brine (100 mL) yielding (9H-fluoren-9-yl)methyl ((4-(2-((6-((4,6-bis(heptadecan-9-yloxy)-1,3,5-triazin-2-yl)amino)hexyl)amino)-2-oxoethoxy)phenyl)(2,4-dimethoxyphenyl)methyl) carbamate.

### Example 2: Peptide synthesis in solution

### Deprotection

The Fmoc protected tag (1 eq.) was dissolved in ethyl acetate (1 %). Then diethylamine (4 eq.) was added and stirred overnight.

### Coupling

The deprotected tag was dissolved in ethyl acetate (1 %). Then the Fmoc protected amino acid (Fmoc-AA-OH) (2 eq) and Oxyma (2 ep) was added and stirred for 1 min. Then N,N'-Diisopropylcarbodiimide (3 eq) was added and stirred overnight.

### Extraction

The reaction mixture was transferred into a separation funnel and washed with water (10 volume eq) and brine (10 volume eq). The tag stays in the organic phase and can be used for the next coupling step in solution.

The solvent was then removed under reduced pressure.

## Claims

1. A compound according to formula (I) 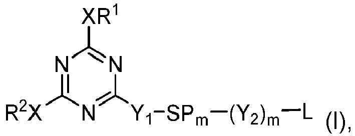 wherein,
X is selected from the group consisting of -O-(CH₂)ₙ-, -NH-(CH₂)ₙ-,-(NMe)-(CH₂)ₙ-, or -S-(CH₂)ₙ, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an alkyl substituted C₆- aryl,
Y is a heteroatom, particularly selected from the group consisting of -O- and -NH-,
L is a linker for peptide synthesis, and
m is 0 or 1, particularly m is 1.

2. The compound according to claim 1, wherein X is selected from -O-(CH₂)ₙ- and -S-(CH₂)ₙ, wherein n is 0 to 4, particularly, wherein X is selected from -O-(CH₂)ₙ-, wherein n is 0 to 4.

3. The compounds according to any of the preceding claims, wherein n is 0.

4. The compound according to claims 1 or 2, wherein R¹ and R² are independently from each other a branched or substituted C₁₁-C₂₅ alkyl.

5. The compound according to any of the preceding claims, wherein R¹ and R² are independently selected from the group consisting of C₁₃-C₂₅ alkyl.

6. The compound according to any of the preceding claims, wherein R¹ is selected from the group consisting of C₁₇-C₂₅ alkyl.

7. The compound according to any of the preceding claims, wherein R² is selected from the group consisting of C₁₇-C₂₅ alkyl.

8. The compound according to any of the preceding claims, wherein Y₁ is -NH- and Y₂ is -NH- or-O.

9. The compound according to any of the preceding claims, wherein Y₁ is -NH- and Y₂ is -NH-.

10. The compound according to any of the preceding claims, wherein SP is selected from the group consisting of C₅-C₇ alkyl.

11. The compound according to any of the preceding claims, wherein the linker L selected from the group consisting of Rink, Sieber, Trityl, Ramage, Wang, Sasrin, HMPB, 2-chlorotrityl and MBHA.

12. The compound according to any of the preceding claims, wherein the linker L is selected from the group consisting of Rink, Sieber and Trityl.

13. An intermediate according to formula (II) 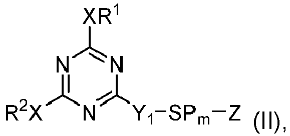 wherein,
X is selected from the group consisting of -O-(CH₂)ₙ-, -NH-(CH₂)ₙ-,-(NMe)-(CH₂)ₙ-, or -S-(CH₂)ₙ-, wherein n is 0 to 4,
R¹ and R² are independently selected from the group consisting of C₁₁-C₂₅ alkyl,
SP is a spacer, particularly selected from the group consisting of saturated C₄-C₈ alkyl, unsaturated C₄-C₈ alkyl and substituted or unsubstituted C₆-aromatic compound, in particular the substituted C₆-aromatic compound is an alkyl substituted C₆- aryl,
Y is a heteroatom, particularly selected from the group consisting of -O- and -NH-,
Z is selected from -OH, -NH₂, and
m is 0 or 1, particularly m is 1.
